# EUROPEAN PATENT SPECIFICATION

(11) **EP 3 460 476 B1**
(45) Date of publication and mention of the grant of the patent: **08.12.2021**
(21) Application number: 17799620.4
(22) Date of filing: 16.05.2017
(51) Int. Cl.: G01N 33/574, C12Q 1/68, A61K 48/00, A61K 39/395, G01N 33/92, C07K 16/28

(54) **BIOMARKER COMPOSITION COMPRISING LRP-1 AS ACTIVE INGREDIENT, FOR DIAGNOSIS OF RADIATION-RESISTANT CANCER OR PREDICTION OF RADIATION THERAPY PROGNOSIS**
BIOMARKERZUSAMMENSETZUNG MIT LRP-1 ALS WIRKSTOFF ZUR DIAGNOSE VON STRAHLUNGSRESISTENTEM KREBS ODER VORHERSAGE EINER STRAHLENTHERAPIEPROGNOSE
COMPOSITION DE BIOMARQUEUR COMPRENANT LRP-1 EN TANT QUE PRINCIPE ACTIF POUR LE DIAGNOSTIC DU CANCER RÉSISTANT À L'IRRADIATION OU LA PRÉDICTION DU PRONOSTIC D'UNE RADIOTHÉRAPIE

(30) Priority: 17.05.2016 KR 20160060323; 15.05.2017 KR 20170060005
(43) Date of publication of application: 27.03.2019
(73) Proprietor: University Of Ulsan Foundation For Industry Cooperation, Ulsan 44610 (KR); The Asan Foundation, Seoul 05505 (KR)
(72) Inventor: CHOI, Eun Kyung, Seoul 05510 (KR); JEONG, Seong-Yun, Yongin-si Gyeonggi-do 16861 (KR); SONG, Si Yeol, Seoul 06321 (KR); LEE, Kyoung Jin, Seoul 02871 (KR)
(74) Representative: Roos, Peter
(86) International application number: PCT/KR2017/005054
(87) International publication number: WO 2017/200263

(56) References cited:
- WO-A1-2011/054644
- WO-A1-2012/115885
- WO-A1-2016/080632
- KR-A- 20140 094 765
- US-A1- 2006 037 088
- US-A1- 2015 079 078
- H. MENG ET AL: "Stromal LRP1 in Lung Adenocarcinoma Predicts Clinical Outcome", CLINICAL CANCER RESEARCH, vol. 17, no. 8, 15 February 2011 (2011-02-15), pages 2426-2433, XP055656986, US ISSN: 1078-0432, DOI: 10.1158/1078-0432.CCR-10-2385
- KIM, MI-HYOUNG et al.: "Quantitative Proteomic Analysis of Single or Fractionated Radiation-induced Proteins in Human Breast Cancer MDA-MB-231 Cells", Cell & Bioscience, vol. 5, no. 2, 2015, pages 1-11, XP021217840,
- S AHLBERG, SARA HAGGBLAD et al.: "Evaluation of Cancer Stem Cell Markers CD 133, CD 44, CD 24: Association with AKT Isoforms and Radiation Resistance in Colon Cancer Cells", PloS One, vol. 9, no. 4, 2014, pages 1-12, XP055200303,
- MUNAKATA, KOJI et al.: "SCGB2A1 Is a Novel Prognostic Marker for Colorectal Cancer Associated with Chemoresistance and Radioresistance", International Journal of Oncology, vol. 44, 2014, pages 1521-1528, XP055556187,

## Description

### [Technical Field]

The present invention relates to a method for in vitro diagnosing radiation-resistant colon cancer. In addition, the present invention relates to an in vitro method of screening a radiation sensitive enhancer for colon cancer cells.

### [Background Art]

A cell which is the smallest unit for making up the human body, maintains balance of the number of the cell, while dividing by intracellular regulatory functions, growing, dying and disappearing when it is normal. When a cell is damaged for some reason, it is treated and restored to serve as a normal cell, but if it does not recover, it will die by itself. However, cancer is defined as a condition in which abnormal cells that cannot control this proliferation and inhibition, are not only excessively proliferated but also invade surrounding tissues and organs, resulting in mass formation and normal tissue destruction for a various reasons. Cancer refers to proliferation of a cell which cannot be inhibited, and destroys the structure and function of normal cells and organs, and thus its diagnosis and treatment are very important. WO 2012/115885 A1 relates to methods and compositions for characterizing a phenotype by analyzing a vesicle, such as a vesicle present in a biological sample derived from a subject's cell. H. MENG ET AL, "Stromal LRP1 in Lung Adenocarcinoma Predicts Clinical Outcome", CLINICAL CANCER RESEARCH, US, (20110215), vol. 17, no. 8, pages 2426 - 2433 relates to a study in which LRP1 mRNA levels were determined in lung tumors from several large, multicenter studies. WO 2011/054644 A1 relates to a nucleic acid molecule comprising an alternative exon of low density lipoprotein-related protein 1 (LRP1), a protein encoded by said nucleic acid molecule and fragments of said nucleic acid and said protein. SAHLBERG ET AL ("Evaluation of Cancer Stem Cell Markers CD133, CD44, CD24: Association with AKT Isoforms and Radiation Resistance in Colon Cancer Cells", PloS One (20140401), vol. 9, issue 4, e94621, inner pages 1-12) describe CD133, CD144 and CD24 as suitable markers to evaluate radiation resistance in colon cancer cells.

Meanwhile, due to genetic differences in individual cancer-causing patients, treatment response is different and cases of treatment for each patient are different thereby having a problem in treatment. Therefore, in order to effectively treat cancer patients, it is required to develop a cancer micro-environment that changes according to the radiation reactivity and a biomarker according to the same, so that a customized diagnosis and treatment for individual patients can be realized.

### [Disclosure]

### [Technical Problem]

The present disclosure provides a biomarker composition for diagnosing radiation-resistant cancer, comprising a LRP-1 as an active ingredient, a composition for diagnosing radiation-resistant cancer comprising an agent capable of measuring expression level of LRP-1 as an active ingredient, a method of radiation-resistant cancer using the same, a pharmaceutical composition for promoting radiation sensitivity to cancer cells comprising LRP-1 protein expression inhibitor or activity inhibitor as an active ingredient, and a method of screening a radiation sensitivity enhancer for cancer cells by measuring expression level of LRP-1 protein

In addition, the present disclosure provides a biomarker composition for predicting radiation therapy prognosis for cancer patients comprising LRP-1 as an active ingredient, a composition for predicting radiation therapy prognosis in cancer patients, comprising a preparation capable of measuring expression level of LRP-1 as an active ingredient, and a method of predicting radiation therapy prognosis in cancer patients using the same.

### [Technical Solution]

In order to solve the above problems, the present invention provides a method for in vitro diagnosing radiation-resistant colon cancer as defined in claim 1.

Also, the present invention provides an in vitro method of screening a radiation sensitivity enhancer for colon cancer cells as defined in claim 2.

### [Advantageous Effects]

Described herein is a biomarker composition for diagnosing radiation-resistant cancer or for predicting radiation therapy prognosis, which comprises LRP-1 as an active ingredient. The present invention investigated LRP-1, a binding partner protein to which a specific peptide sequence specifically targeting to a radiation-resistant colon cancer tissue is actually bound, and suggested the possibility as a factor related with radiation therapy resistance of colon cancer or a factor for screening a radiation sensitivity enhancer for colon cancer cells.

### [Description of Drawings]

FIG. 1 shows the results of biopanning progression in a human-derived colon cancer cDNA library. The biopanning process was performed three times in total, and it was confirmed that the number of phages was increased specifically as the number of times increased.
FIG. 2 shows a schematic diagram of the construction of a mouse model transplanted with colon cancer tissue of patient.
FIG. 3 shows the results of verifying 7 candidate groups showing significant mRNA expression differences among 14 binding partner protein candidates which are screened.
FIG. 4 shows the results of confirming the expression difference at the protein level of the two candidate groups selected in the confirmation of mRNA expression difference according to irradiation. Only LRP-1 of the two candidate groups identified the expression difference and only the results are shown. For radiation resistance and sensitivity in each case, the expression of the protein was determined according to the irradiation.
FIG. 5 shows the results of histological test of LRP-1 in radiation-resistant and sensitive colon cancer.

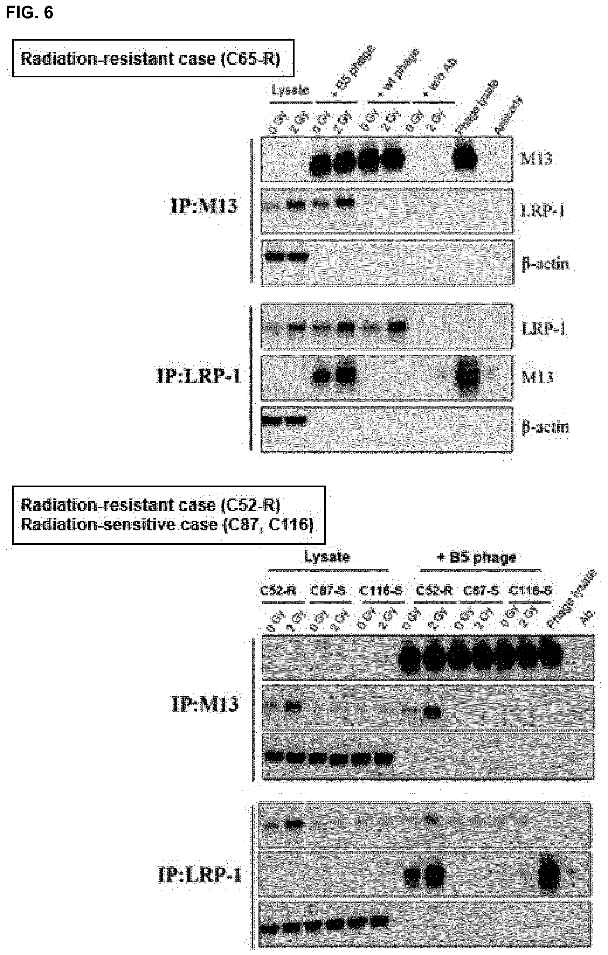

FIG. 6 shows the results of binding between LRP-1 and TPSFSKI peptides using the immunoprecipitation method. Lysate: 0 Gy/2 Gy protein extracted from radiation colon cancer tissue, IP (B5 phage): 0 Gy/2 Gy Immunoprecipitation with a specific peptide phage in protein extracted from radiation colon cancer tissue, IP (wt phage): 0 Gy/2 Gy Immunoprecipitation in protein extracted from radiation colon cancer tissue with phage without peptide, IP (w/o Ab): 0 Gy/2 Gy Immunoprecipitation without antibody in protein extracted from radiation colon cancer tissue, phage lysate: protein extracted from peptide phage targeting by irradiation in radiation resistant colon cancer tissue, Antibody: LRP-1 antibody
FIG. 7 shows the results of confirming the expression of LRP-1 in four cases of colon cancer tissues of a real patient (two cases of radiation resistance and two cases of radiation sensitivity). It was confirmed that the cancer tissue was the same as the cancer tissue extirpate from the mouse model transplanted with patient's cancer tissue, and that LRP-1 was overexpressed in the radiation-resistant case, but was not in the radiation-sensitive case.
FIG. 8 shows the results of verifying the expression of LRP-1 by receiving cancer tissues from 20 patients with poor prognosis of treatment, who underwent radiation therapy among colon cancer patients. It was confirmed that LRP-1 was clearly overexpressed in cancer tissues of patients with poor prognosis for radiation therapy.
FIG. 9 shows the results of *in-silico* analysis of confirming prognosis of patients who underwent radiation therapy among real patients with colon cancer. It was confirmed by clinical data that the radiation therapy prognosis was poor when LRP-1 expression was high.

### [Best Mode]

Described herein is a biomarker composition for diagnosing radiation-resistant cancer, comprising a low density lipoprotein receptor-related protein 1 (LRP-1) or a gene encoding the same, as an active ingredient.

The "low density lipoprotein receptor-related protein 1 (LRP-1)" of the present invention may be NCBI accession no. NM_002332, but It is not limited thereto.

Preferably, the biomarker composition may further comprise a known radiation-resistant biomarker, and the known radiation-resistant biomarker includes CD133, CD144 and CD24, but it is not limited thereto.

Preferably, the LRP-1 can bind to a peptide (TPSFSKI) composed of amino acid sequence of SEQ ID NO: 1, wherein the "TPSFSKI" peptide is a peptide targeting radiation-resistant colon cancer and is disclosed in detail in Korean patent application No. 10-2015-0106580 (related to patent KR20160059944 A).

As used herein, the term "diagnosis" includes determining the susceptibility of an object to a particular disease or disorder, determining whether an object currently has a particular disease or disorder.

Also described herein is a composition for diagnosing radiation-resistant cancer comprising an agent capable of measuring expression level of LRP-1 as an active ingredient.

Specifically, the agent capable of measuring expression level of LRP-1 may be a primer or a probe specifically binding to a gene of the LRP-1, an antibody, a peptide, an aptamer or a compound, which is specifically binding to a protein of the LRP-1, but it is not limited thereto.

Further described herein is a kit for diagnosing radiation-resistant cancer comprising composition.

As used herein, the term "primer" refers to a nucleic acid sequence having a short free 3' hydroxyl group, which can form base pairs with a complementary template, and short nucleic acid serving as a starting point for template strand replication. Primers can initiate DNA synthesis in the presence of reagents for polymerization (i.e., DNA polymerase or reverse transcriptase) and four different nucleoside triphosphates under appropriate buffer solutions and temperatures. The PCR conditions the lengths of the sense and antisense primers can be appropriately selected according to techniques known in the art.

As used herein, the term "probe" refers to a nucleic acid fragment such as RNA or DNA corresponding to a few base or several hundreds of bases that can specifically bind to an mRNA and the presence or absence of a specific mRNA, expression level can be confirmed by labeling. The probe may be prepared in the form of an oligonucleotide probe, a single strand DNA probe, a double strand DNA probe, or an RNA probe. Selection of suitable probes and hybridization conditions can be appropriately selected according to techniques known in the art.

As used herein, the term "antibody" is well known in the art and means a specific immunoglobulin as directed against an antigenic site. An antibody in the present invention means an antibody which specifically binds to LRP-1 of the present invention, and an antibody can be produced according to a conventional method in the art. The form of the antibody includes polyclonal or monoclonal antibodies, including all immunoglobulin antibodies. The antibody refers to a complete form having two full-length light chains and two full-length heavy chains. The antibody also includes a special antibody such as a humanized antibody.

The kit may further comprise an antibody specifically binding to the marker component, a secondary antibody conjugate conjugated with a labeling substance color-developed by the reaction with the substrate, a coloring substrate solution to be colored with the labeling substance, washing liquid, an enzyme reaction stop solution, and the like, and may be manufactured as a number of separate packaging or compartments including the reagent components used.

As used herein, the term "peptide" has a high binding capacity to a target material and does not cause denaturation during thermal/chemical treatment. Also, because of its small size, it can be used as a fusion protein by attaching it to other proteins. It can be used as a diagnostic kit and a drug delivery material because it can be specifically attached to a polymer protein chain.

As used herein, the term "aptamer" refers to a polynucleotide composed of a specific type of single-stranded nucleic acid (DNA, RNA or modified nucleic acid) having a stable tertiary structure by itself and having the property for capable of binding to a target molecule with high affinity and specificity. As described above, since the aptamer is composed of a polynucleotide which can specifically bind to an antigenic substance like an antibody and is more stable than the protein, has a simple structure, and is easy to synthesize and thus can be used instead of an antibody.

The present invention provides a method for in vitro diagnosing radiation-resistant colon cancer as defined in claim 1, the method comprising: (1) measuring mRNA expression level of LRP-1 gene or expression level of LRP-1 protein from a sample isolated from a colon cancer patient; (2) comparing the mRNA expression level of the LRP-1 gene or the expression level of the LRP-1 protein with a control sample; and (3) determining that the colon cancer is a radiation-resistant colon cancer when the mRNA expression level of the LRP-1 gene or the expression level of the LRP-1 protein is higher than that of the control sample.

Specifically, the method of measuring the mRNA expression level may be RT-PCR, competitive RT-PCR, real-time RT-PCR, RNase protection assay, Northern blotting and DNA chips, but it is not limited thereto.

Specifically, the method of measuring the protein expression level may be Western blot, enzyme linked immunosorbent assay (ELISA), radioimmunoassay (RIA), radioimmunodiffusion, Ouchterlony immunodiffusion, rocket, immunoelectrophoresis, tissue immunostaining, immunoprecipitation assays, complement fixation assays, FACS and protein chips, but it is not limited thereto.

As used herein, the term "sample isolated from a cancer patient" includes a sample such as tissue, cell, whole blood, serum, plasma, saliva, sputum, cerebrospinal fluid, or urine, which is different from the control group in the expression level of the LRP-1 gene or the LRP-1 protein, a biomarker for diagnosing radiation-resistant cancer, but it is not limited thereto.

In the present invention, the term "radiation-resistant cancer diagnosis" is intended to confirm whether cancer cells are resistant or susceptible to radiation.

Also described herein is a pharmaceutical composition for promoting radiation sensitivity to cancer cells comprising LRP-1 protein expression inhibitor or activity inhibitor as an active ingredient.

Specifically, the LRP-1 protein expression inhibitor may be an antisense nucleotide complementary to mRNA of LRP-1 gene, a small interfering RNA (siRNA) or a short hairpin RNA (shRNA) and the LRP-1 protein activity inhibitor may be a compound, a peptide, a peptide mimetic, an aptamer, an antibody or natural products, which specifically binds to an LRP-1 protein.

The pharmaceutical composition used may contain a chemical substance, a nucleotide, an antisense, an siRNA oligonucleotide and a natural product extract as an active ingredient. The pharmaceutical composition or combination preparation may be prepared by using pharmaceutically acceptable and physiologically acceptable adjuvants in addition to the active ingredients, and examples of the adjuvants include solubilizers such as excipients, disintegrants, sweeteners, binders, coating agents, swelling agents, lubricants, slip modifiers or flavors. The pharmaceutical composition may be formulated into a pharmaceutical composition containing at least one pharmaceutically acceptable carrier in addition to the active ingredient for administration. Acceptable pharmaceutical carriers for compositions which are formulated into liquid solutions include sterile saline, sterile water, Ringer's solution, buffered saline, albumin injection solution, dextrose solution, maltodextrin solution, glycerol, ethanol and its mixture of at least one, which is suitable for sterilization and in vivo, and if necessary, other conventional additives such as an antioxidant, a buffer, and a bacteriostatic agent may be added. In addition, diluents, dispersants, surfactants, binders, and lubricants can be additionally added to formulate into injectable solutions such as aqueous solutions, suspensions, emulsions and the like, pills, capsules, granules or tablets.

The pharmaceutical preparation form of the pharmaceutical composition may be granules, powders, coated tablets, tablets capsules, suppositories, syrups, juices, suspensions, emulsions, drips or injectable liquids and a sustained release formulation of the active compound, and the like. The pharmaceutical compositions may be administered in a conventional manner via Intravenous, intraarterial, intraperitoneal, intramuscular, intraarterial, intraperitoneal, intrasternal, percutaneous, nasal, inhaled, topical, rectal, oral, intraocular or intradermal routes. The effective amount of the active ingredient of the pharmaceutical composition means the amount required for preventing or treating the disease. Accordingly, the present aspects can be adjusted according to various factors such as the particular type of the disease, the severity of the disease, the kind and amount of the active ingredient and other ingredients contained in the composition, the type of formulation and the patient's age, body weight, general health status, sex and diet, time of administration, route of administration and ratio of the composition, duration of treatment, concurrent medication, and the like, but it is not limited thereto. For example, in the case of an adult, when administered once to several times a day, the composition of the present invention may be administered at a dose of 0.1 ng/kg to 10 g/kg of compound, 0.1 ng/kg to 10 g/kg of In protein or antibody, 0.01 ng/kg to 10 g/kg of antisense nucleotide, siRNA, shRNAi and miRNA.

In addition, the present invention provides an in vitro method of screening a radiation sensitivity enhancer for colon cancer cells as defined in claim 2, the method comprising: (1) contacting a test substance with a colon cancer cell; (2) measuring a level of expression or activity level of LRP-1 protein in the colon cancer cells in contact with the test substance; and (3) selecting a test substance of which the level of expression or activity of the LRP-1 protein is decreased as compared with that of a control sample.

The term "test substance" used in referring to the screening method of the present invention means an unknown candidate substance used in screening to examine whether it affects the expression amount of a gene or the expression or activity of a protein. The samples include chemicals, nucleotides, antisense-RNA, siRNA (small interference RNA) and natural extracts, but it is not limited thereto.

Further described is a biomarker composition for predicting radiation therapy prognosis for cancer patients comprising LRP-1 or a gene encoding the same as an active ingredient.

As used herein, the term "prognosis prediction" refers to an act of predicting the course and result of a disease beforehand. More specifically, the course of the disease after treatment may vary depending on the physiological or environmental condition of the patient, and it can be interpreted as meaning all the actions that predict the course of the disease after treatment considering the condition of the patient as a whole.

For the purpose of the present disclosure the prognosis prediction can be interpreted as predicting the disease-free survival rate or the survival rate of the cancer patient by predicting the course of the disease and the complete treatment after the radiation therapy of a cancer patient. For example, predicting a "good prognosis" indicates a high level of disease-free survival or survival rate in cancer patients after radiation therapy, which implies that cancer patients are more likely to be treated, and the prediction of "poor prognosis" indicates a low level of disease-free survival or survival rate in cancer patients after radiation therapy, which implies that the cancer is likely to recur from cancer patients or the patients is likely to die due to cancer.

In addition, also described herein is a composition for predicting radiation therapy prognosis in cancer patients, comprising a preparation capable of measuring expression level of LRP-1 as an active ingredient.

Specifically, the agent capable of measuring expression level of LRP-1 may be a primer or a probe specifically binding to a gene of the LRP-1, an antibody, a peptide, an aptamer or a compound, which is specifically binding to a protein of the LRP-1, but it is not limited thereto.

In addition, also described herein is a kit for predicting radiation therapy prognosis in a cancer patient comprising the composition.

In addition, also described herein is a method of providing information necessary for predicting radiation therapy prognosis in cancer patients comprising: (1) measuring mRNA expression level of LRP-1 gene or the LRP-1 protein expression level from a sample isolated from a cancer patient; (2) comparing the mRNA expression level of the LRP-1 gene or the LRP-1 protein expression level with that of a control sample; and (3) determining that the radiation therapy prognosis is poor when the mRNA expression level of the LRP-1 gene or the LRP-1 protein expression level is higher than that of the control sample.

Specifically, the method of measuring the mRNA expression level may be RT-PCR, competitive RT-PCR, real-time RT-PCR, RNase protection assay, Northern blotting and DNA chips, but it is not limited thereto.

Specifically, the method of measuring the protein expression level may be Western blot, enzyme linked immunosorbent assay (ELISA), radioimmunoassay (RIA), radioimmunodiffusion, Ouchterlony immunodiffusion, rocket, immunoelectrophoresis, tissue immunostaining, immunoprecipitation assays, complement fixation assays, FACS and protein chips, but it is not limited thereto.

In the present disclosure, the term "cancer" or "cancer cell" may be breast cancer, cervical cancer, glioma, brain cancer, melanoma, lung cancer, bladder cancer, prostate cancer, leukemia, renal cancer, liver cancer, colon cancer, pancreatic cancer, stomach cancer, gallbladder cancer, ovarian cancer, lymphoma, osteosarcoma, uterine cancer, oral cancer, bronchial cancer, nasopharyngeal cancer, laryngeal cancer, skin cancer, blood cancer, thyroid cancer, parathyroid cancer or ureteral cancer, or its cancer cell, but it is limited thereto.

Hereinafter, the present invention will be described in detail with reference to the following examples. It should be noted, however, that the following examples are illustrative of the present invention and are not intended to limit the scope of the present invention. The examples of the present invention are provided to more fully describe the present invention to those skilled in the art.

### <Example 1> Screening of human colon cancer-derived cDNA library

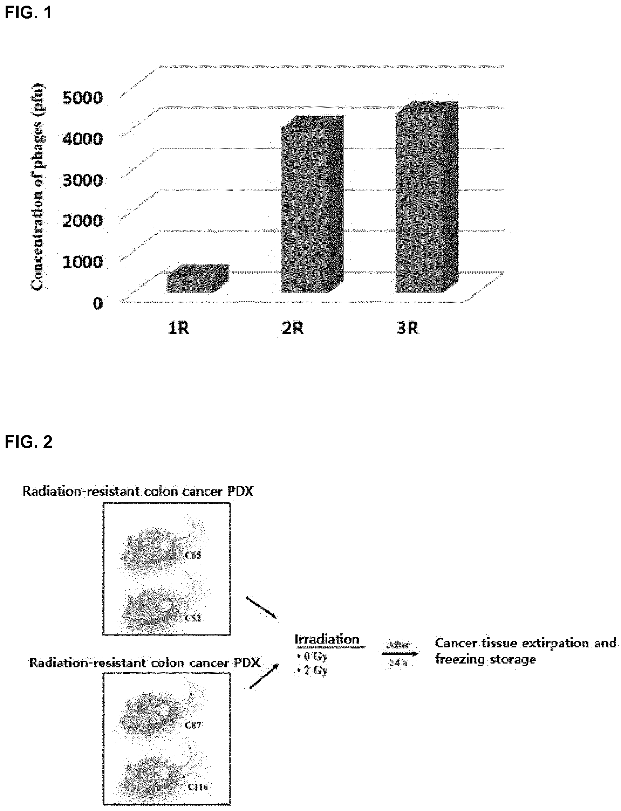

T7Select^{®} Human colon tumor cDNA library (Novagen) was purchased and screened for binding partner proteins of a specific peptide sequence (TPSFSKI; Korean patent application No. 10-2015-0106580) targeting radiation-resistant colon cancer. Specifically, the biopanning process was repeated three times to design a candidate group for binding partner protein, and a candidate group that specifically binds was selected. After completing total three bio panning procedures, possible candidate group information was obtained using sequence analysis and BLAST. The information of candidate group was shown in FIG. 1.

### <Example 2> Construction of mouse model of patient colon cancer tissue transplantation

Radiation-resistant case tissues among patient colon cancer tissues were subcultured in nude mice. One cancer tissue was implanted subcutaneously under the thigh, respectively. When the size of the cancer tissue grew to about 100 mm³ by about 1 month (4 weeks) during the observation of growth of cancer tissue, 2 Gy of radiation was irradiated locally to only the cancer tissue and it was used for experiment after 24 hours of recovery. As a control group of the irradiation group, a mouse model not irradiated alone (0 Gy) was also prepared in the same mouse model. In addition, not only the radiation-sensitive colon cancer case but also the radiation-sensitive colon cancer case were transplanted and subcultured in the same manner, and the irradiation was also carried out under the same conditions. A schematic diagram related to the construction of a mouse model was shown in FIG. 2.

### <Example 3> Confirmation of significant mRNA expression difference in candidate group of binding partner protein

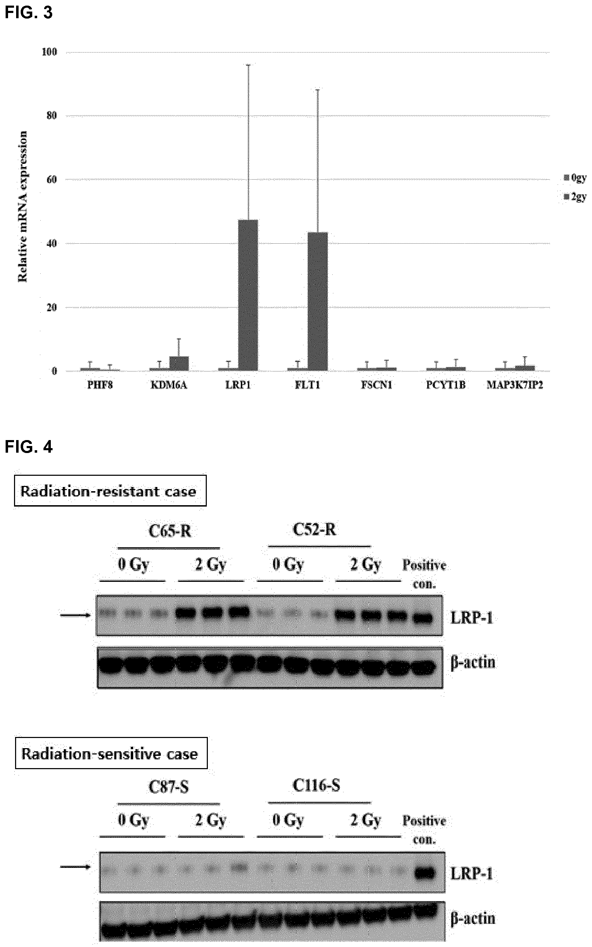

For selecting and validating candidate group of binding partner protein, the Q-PCR technique was used to identify the significant mRNA expression differences according to radiation sensitive and resistant colon cancer cases and irradiation. Primers for 14 binding partner protein candidate genes obtained by screening were prepared. In addition, cancer tissues were extirpated from the model constructed in Example 2, and followed by freezing, mRNAs were purified and cDNAs were synthesized. Quantitative PCR (Q-PCR) was carried out using the same and 7 candidate groups showing significant difference in expression among 14 candidate groups were selected according to irradiation. As a result of confirming the reproducibility of the candidate group, two candidate groups had the largest significant expression difference and the results were shown in FIG. 3.

### <Example 4> Confirmation of protein expression difference according to radiation Irradiation of binding partner protein candidates

The western blotting technique was used to prove significant expression differences at the protein level for the two candidates verified according to the significant difference in mRNA expression. Protein was extracted from the frozen tissues and the protein expression difference by irradiation was confirmed using the antibody of each candidate group. Of the two candidates, only LRP-1 could verify significant protein expression differences according to radiation irradiation, and one of the other candidates was excluded from the candidate group because the protein level could not be determined regardless of the irradiation. In addition to the radiation-resistant colon cancer cases used in the screening and continue verification steps, two additional cases of radiation-sensitive colon cancer and one case of radiation-resistant colon cancer were added to confirm the protein expression difference. In the case of LRP-1, significant expression difference according to radiation irradiation was not observed in the case of radiation-sensitive cases, and in case of other radiation-resistant colon cancer cases, a significant expression difference was observed according to irradiation again. The results were shown in FIG. 4.

### <Example 5> Histological verification of LRP-1

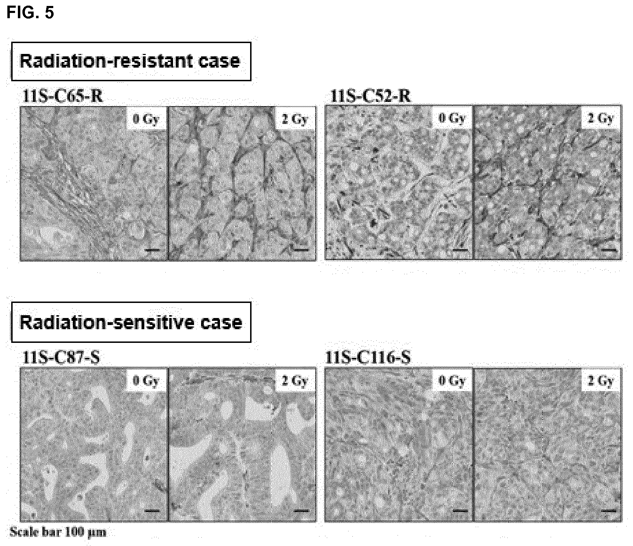

IHC staining technique was performed for histological verification of LRP-1 which showed significant expression difference at mRNA and protein level. Radiation-resistant and susceptible colon cancer tissue sections were prepared by irradiation as two respective cases and LRP-1 antibody was used to stain the tissue sections and the existence of the sections was verified. By confirming the homology with the protein expression difference in Example 4, LRP-1 is a binding partner protein of TPSFSKI peptide. It was shown in FIG. 5.

### <Example 6> Identification of actual binding of TPSFSKI peptide phage and LRP-1

Based on the indirect verification that LRP-1 is a binding partner protein of the TPSFSKI peptide sequence in Examples 3 to 5, an immunoprecipitation assay was performed to directly determine whether LPS-1 was bound to the TPSFSKI sequence. Proteins were extracted from colon cancer tissues of each case according to radiation irradiation, and immunoprecipitation was performed using LRP-1 and M13 antibodies and IgG beads. Immunoprecipitation was confirmed by western blotting, which directly confirmed that LRP-1 is a binding partner protein of TPSFSKI peptide. The results were shown in FIG. 6.

### <Example 7> Confirmation of expression of LRP-1 in actual patient colon cancer tissue

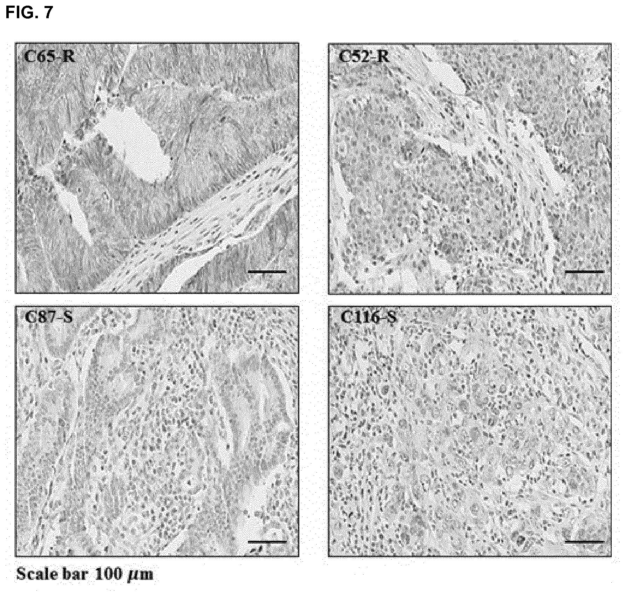

The cancer tissues utilized in Examples 4 to 6 are cancer tissues extirpated from a patient colon cancer tissue transplantation mouse model. The expression of LRP-1 and the expression difference of each case were verified in the patient's colon cancer tissue, which is the original host of the cancer tissue. With the approval of the relevant institution, real cancer tissue paraffin slice slide of the patient was provided from the pathology department of Asan Medical Center in Seoul, Korea and histological verification was carried out by the IHC staining technique. Thus, results correlated to Example 4 and FIG. 5 were obtained, and it was verified that LRP-1 can be applied in actual clinical practice. The results were shown in FIG. 7.

### <Example 8> Verification of expression of LRP-1 in cancer tissue of radiation-resistant colon cancer patients

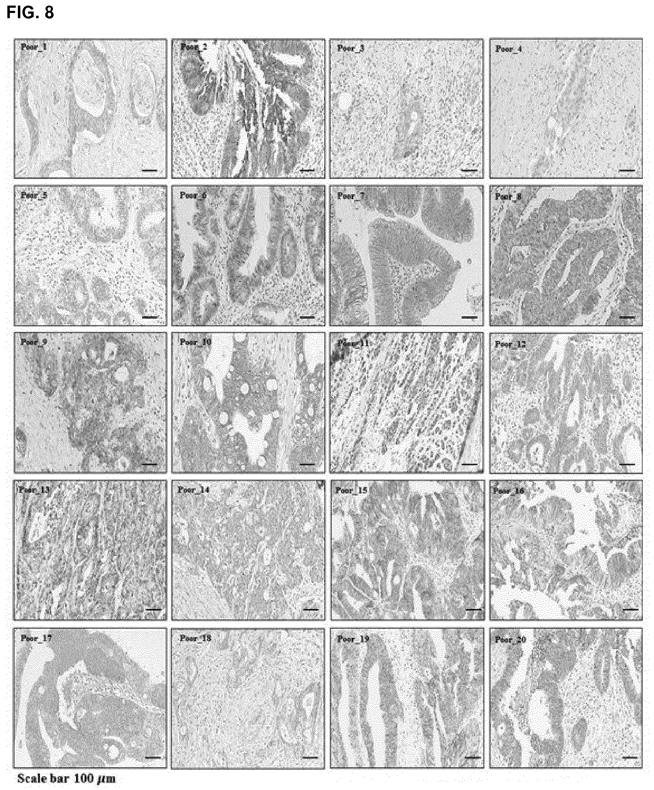

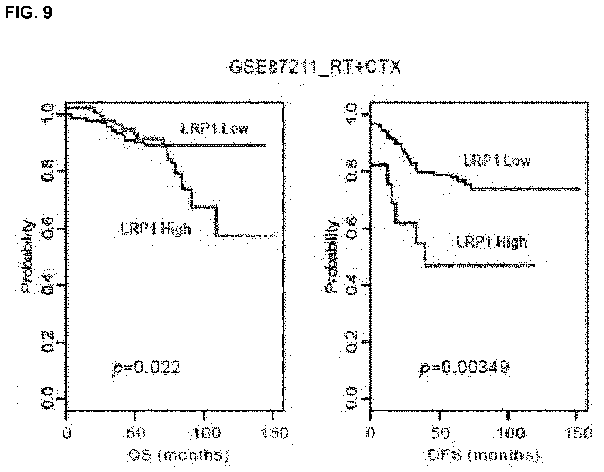

In addition to Example 7, the expression of LRP-1 was verified in 20 colon cancer tissue cases of patients with poor prognosis of radiation therapy among patients who underwent actual radiation therapy. The verification method was performed by the IHC staining technique in the same manner as in Example 7, and as a result, it was confirmed that LRP-1 was overexpressed in colon cancer tissue of patients with poor prognosis of irradiation therapy. In addition, the prognosis according to the expression of LRP-1 was analyzed in patients with colon cancer who underwent radiation therapy through clinical data analysis. As a result, it was confirmed that the prognosis was very poor when the expression of LRP-1 was high. The results were shown in FIGS. 8 and 9.
## Claims

1. A method for in vitro diagnosing radiation-resistant colon cancer comprising:
(1) measuring mRNA expression level of LRP-1 gene or expression level of LRP-1 protein from a sample isolated from a colon cancer patient;
(2) comparing the mRNA expression level of the LRP-1 gene or the expression level of the LRP-1 protein with a control sample; and
(3) determining that the colon cancer is a radiation-resistant colon cancer when the mRNA expression level of the LRP-1 gene or the expression level of the LRP-1 protein is higher than that of the control sample.

2. An in vitro method of screening a radiation sensitivity enhancer for colon cancer cells comprising:
(1) contacting a test substance with a colon cancer cell;
(2) measuring a level of expression or activity level of LRP-1 protein in the colon cancer cells in contact with the test substance; and
(3) selecting a test substance of which the level of expression or activity of the LRP-1 protein is decreased as compared with that of a control sample.

## Patentansprüche

1. Verfahren zur In-vitro-Diagnose von strahlenresistentem Darmkrebs, umfassend:
(1) Messen des mRNA-Expressionsniveaus des LRP-1-Gens oder des Expressionsniveaus des LRP-1-Proteins aus einer von einem Darmkrebspatienten isolierten Probe;
(2) Vergleichen des mRNA-Expressionsniveaus des LRP-1-Gens oder des Expressionsniveaus des LRP-1-Proteins mit einer Kontrollprobe; und
(3) Bestimmen, dass der Darmkrebs ein strahlungsresistenter Darmkrebs ist, wenn das mRNA-Expressionsniveau des LRP-1-Gens oder das Expressionsniveau des LRP-1-Proteins höher ist als das der Kontrollprobe.

2. In vitro-Verfahren zum Screening eines eines die Strahlungsempfindlichkeit erhöhenden Mittels für Darmkrebszellen, umfassend:
(1) Kontaktieren einer Testsubstanz mit Darmkrebszellen;
(2) Messen des Expressions- oder Aktivitätsniveaus des LRP-1-Proteins in den Darmkrebszellen, die mit der Testsubstanz in Kontakt sind; und
(3) Auswählen einer Testsubstanz, bei der das Expressions- oder Aktivitätsniveau des LRP-1-Proteins im Vergleich zu dem einer Kontrollprobe verringert ist.

## Revendications

1. Procédé pour le diagnostic in vitro du cancer du côlon radioresistant, comprenant :
(1) mesurer le niveau d'expression de l'ARNm du gène LRP-1 ou le niveau d'expression de la protéine LRP-1 à partir d'un échantillon isolé d'un patient du cancer du côlon ;
(2) comparer le niveau d'expression de l'ARNm du gène LRP-1 ou le niveau d'expression de la protéine LRP-1 avec un échantillon témoin ; et
(3) déterminer que le cancer du côlon est un cancer du côlon radiorésistant lorsque le niveau d'expression de l'ARNm du gène LRP-1 ou le niveau d'expression de la protéine LRP-1 est supérieur à celui de de l'échantillon témoin.

2. Procédé in vitro de criblage d'un amplificateur de radiosensibilité des cellules du cancer du côlon comprenant :
(1) contacter une substance d'essai avec des cellules cancéreuses du côlon ;
(2) mesurer un niveau d'expression ou un niveau d'activité de la protéine LRP-1 dans les cellules cancéreuses du côlon en contact avec la substance d'essai ; et
(3) sélectionner une substance d'essai dont le niveau d'expression ou d'activité de la protéine LRP-1 est diminué par rapport à celui d'un échantillon témoin.


<110> University of Ulsan Foundation For Industry Cooperation THE ASAN FOUNDATION
<120> Biomarker composition for diagnosing radiation resistant cancer or predicting prognosis of radiation therapy comprising LRP-1
<130> AOP-2017-0035PCT
<150> KR 10-2016-0060323
   <151> 2016-05-17
<150> KR 10-2017-0060005
   <151> 2017-05-15
<160> 1
<170> KopatentIn 2.0
<210> 1
   <211> 7
   <212> PRT
   <213> human
<400> 1
   Thr Pro Ser Phe Ser Lys Ile
     1               5